# EUROPEAN PATENT SPECIFICATION

(11) **EP 1 610 667 B1**
(45) Date of publication and mention of the grant of the patent: **26.09.2007**
(21) Application number: 03720862.6
(22) Date of filing: 04.04.2003
(51) Int. Cl.: A61B 1/005, A61B 1/04, A61B 1/018

(54) **ENDOSCOPIC INSTRUMENT WITH TWO INDEPENDENT ARMS**
ENDOSKOPISCHES INSTRUMENT MIT ZWEI UNABHÄNGIGEN ARMEN
INSTRUMENT ENDOSCOPIQUE COMPORTANT DEUX BRAS INDEPENDANTS

(43) Date of publication of application: 04.01.2006
(73) Proprietor: Universita Cattolica Del Sacro Cuore, 20123 Milano (IT)
(72) Inventor: Spera, Gianluca, Univ. Cattolica del Sacro Cuore, 00186 ROMA (IT)
(74) Representative: Leone, Mario
(86) International application number: PCT/IT2003/000206
(87) International publication number: WO 2004/086959

(56) References cited:
- US-A- 5 025 778
- US-A- 5 689 365
- US-A1- 2002 055 668
- US-A1- 2002 077 546

## Description

The present invention refers to an endoscopic instrument, in particular to an advanced endoscope particularly useful for the non-invasive treatment of some digestive system pathologies.

To date, nearly every endoscopic instrument is made according to a common and time-honored architecture.

Image management has evolved, also due to the normal taking over of new technologies, however instrument mechanics and architecture remained the same.

Front vision endoscopes (e.g. the common gastroscope or the colonscope), and side vision instruments (the duodenoscope) are known.

Besides from the above, miniaturized endoscopes (e.g. cholangioscope) in all reproducing the latter were made.

Common denominator of all known instruments for digestive endoscopy is the presence of a single working channel (it also having a front or side exit) always having a direction consensual to the instrument body. In fact, such channel is internal to the endoscope body and passively submitting to its motions. The working channel allows the use of tools and/or of other accessories like pincers, balloons, extraction baskets or lancets for carrying out therapeutic maneuvers.

USA Patent n. 5,025,778 relates to an endoscope with a plurality of working channels and a vision apparatus. The working arms inserted in the working channels, can move according a longitudinal direction and can operate in a cooperative relationship. Nevertheless, the arms of such device cannot operate complex movements and for such reason the use of device does not result in a realistic reproduction of the classical surgery technique.

To date, the therapeutic scope of digestive endoscopy covers treatments like, e.g. the draining of biliary lithiasis, the treatment of chronic pancreatitis, the palliation of biliary tract neoplastic stenoses by positioning prostheses, the resection of incipient neoplastic transformation areas (e.g. mucosectomy) or of adenomatous areas (e.g. ampullectomy, polipectomy).

To date, also the treatment of bleeding, the dilation of benign stenoses and the reduction of gastroesophageal reflux are carried out by endoscopy.

However, the great limitation of operative endoscopy remains that of not allowing the resection of wider areas of organs, as a single working channel having reduced mobility would not allow managing potential complications.

For these and other situations laparoscopic or laparotomic surgery has to be resorted to.

This however increases costs, lengths of hospital stay, and the general discomfort of the patient, who is unable to choose effective non-invasive and conservative techniques.

Object of the present invention is to solve the described known-art problems, providing an endoscopic instrument having a flexible and elongate main body that houses a vision device for taking images of an organ internal area, comprising a first working arm for the use of tools and a second working arm for the use of tools apt to be operated independently with respect to said first working arm, characterized in that said first and second working arm are transversally movable with respect to said main body, moving away and/or nearing the one with respect to the other.

The instrument according to the present invention is an operative endoscopic device having advanced functions and an application scope lying between the endoscopic and the laparoscopic surgery fields.

The main advantage of an instrument according to the present invention, with respect to the known-art endoscopic instruments, is provided by the fact that it allows to endoscopically treat a range of pathologies that at present are treatable only surgically, thereby giving surgical/therapeutic ends to a specialty originally meant to have substantially diagnostic aims.

Hence, an instrument according to the present invention allows to transfer the features and the advantages of some surgical techniques, especially of modern laparoscopy, to the field of endoscopy.

This is made possible mainly by virtue of the employ of two independent working arms that allow laparoscopy-like maneuvers inside of endoscopically investigated organs.

Further advantages, features, and the operation modes of the present invention will be made apparent in the following detailed description of preferred embodiments thereof, given by way of example and not for limitative purposes, making reference to the figures of the annexed drawings, wherein:
figure 1 shows a known-art endoscope;

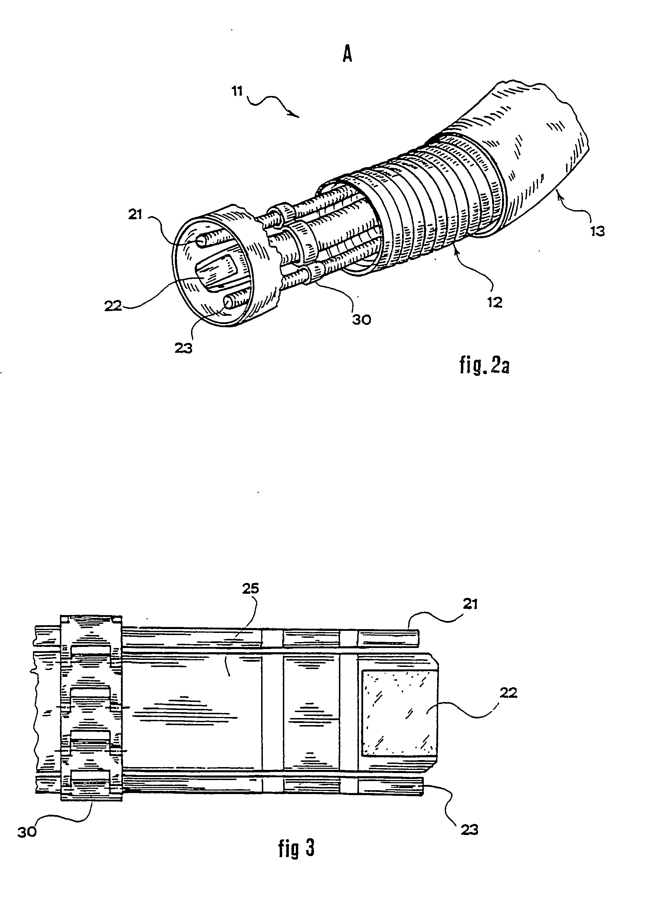

figure 2 is an exemplary sketch of a system for actuating and controlling an instrument according to the present invention;
figure 2A is a cross section of the working end portion of an endoscopic instrument according to the present invention;
figure 3 is a detail of the working end portion of an instrument according to the present invention in a resting position;
figure 4 is a detail of the working end portion with the two working arms extended;
figure 5 is a detail of the working end portion with the two working arms spaced from the central body;
figure 6 is a sketch of an annular mechanism for connecting the working arms;
figure 7 is a detail of the working end portion with the two working arms in a working position;
figure 8 is an exemplary view of the use of an instrument according to the present invention; and
figure 9 is an optional virtual visualization of the path of the instrument in the explored organ.

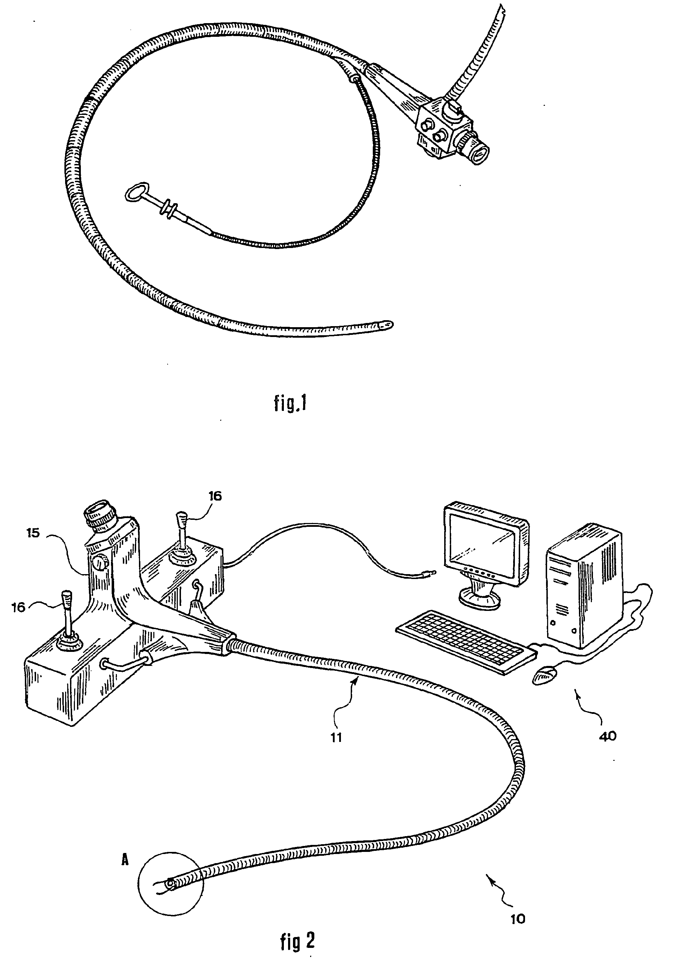

With initial reference to figure 1, it shows a known-art endoscope.

Hence, an endoscope 1 is essentially made of a flexible main body, comprising a working arm and a vision device, and of a manual apparatus 2 for motion control and image viewing.

With reference to the subsequent figures, an endoscopic instrument 10 according to the present invention comprises a flexible and elongate main body 11, having an annular metal skeleton 12 coated with an external sheath 13 preferably obtained from a rubber mix, such as to easily perform spatial motions.

For simplicity's sake, hereinafter reference will be made to an endoscope, this term being meant to refer to an endoscopic instrument according to the present invention.

The handling of the main body 11 is attained by a control system comprising first handling means (not shown in the figures), e.g. a system having one or more tie rods, mechanically operated by a user via controls 15.

Such a tie rod system will not be detailed, since its embodiment is substantially equivalent to those of known-art endoscopes and therefore within the capacity of a person skilled in the art.

Alternatively, or even in association to the tie rod system, such first handling means may advantageously comprise first motion actuating and controlling devices of electronic and/or electromechanical type.

In that case, the motions of the main body will be servoassisted, and the user will control such motions via electronic controls, e.g. a joystick or the like.

Such devices and the corresponding actuating and controlling instruments are directly derivable from the known art, with the evident and required adaptations for the specific applications. Therefore, a detailed description thereof will be omitted, such an embodiment being within the capacity of a person skilled in the art.

The endoscope 10 comprises a central body where there is located a vision device 22, e.g. a camera, preferably a digital camera, for taking images related to the internal area of an organ.

It comprises a first working arm 21 for the use of tools, e.g. pincers, lancets, etc.

According to the present invention, the endoscope 10 further comprises a second working arm 23 for the use of tools in order to work on the examined organ.

The two working arms 21, 23 are both flexible and have a structure substantially analogous to that of the main body, hence having each a respective metal skeleton covered with a flexible rubber sheath.

Advantageously, the two working arms may be handled independently the one from the other.

Second handling means are provided in order to allow predetermined motions to the working arms 21, 23.

As is the case for the main body, such second handling means comprises a system of one or more tie rods, mechanically operated by a user.

Alternatively, or even in association to the tie rod system, such second handling means may advantageously comprise second motion actuating and controlling devices of electronic and/or electromechanical type.

In that case the motions of the two arms will be servoassisted and the user will control such motions via electronic controls, e.g. a pair of joystick 16 or the like.

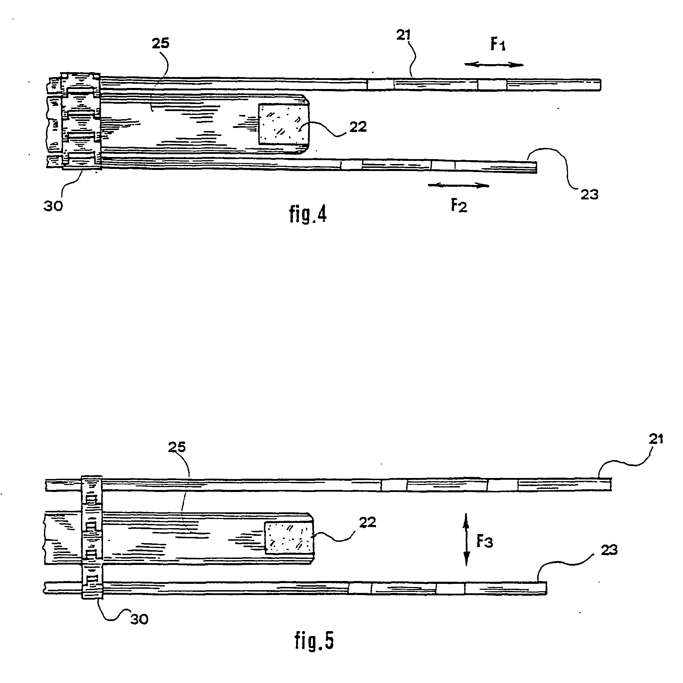

In particular, each of the two working arms may slide longitudinally with respect to the main body, independently the one from the other, as indicated in figure 4 by the arrow F1 for the arm 21, and by the arrow F2 for the arm 23, respectively.

Moreover, the two arms can move transversally with respect to the main body, moving away and/or nearing the one with respect to the other, as it is indicated by arrow F3 of figure 5.

The endoscope further comprises a central body 25, the image-taking camera being connected at the working end thereof.

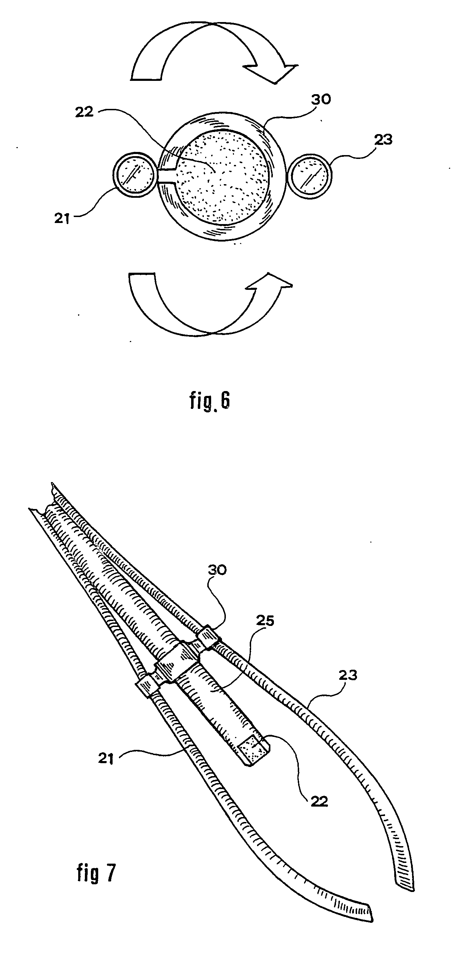

According to the preferred embodiment, the two working arms 21, 23 are located on two opposite sides of said central body and are connected thereto by one or more annular mechanisms 30, each one being apt to rotate about the central body, when suitably controlled by the operator, as it is illustrated in figure 6.

Each such annular mechanisms comprises a pair of slides in order to allow the two working arms to slide longitudinally with respect to the central body.

Such annular mechanisms are made so as to have a certain elasticity allowing also the transversal motions for the moving away/nearing of the two working arms with respect to the central body. This elasticity is attained e.g. through an elastically connected mesh structure.

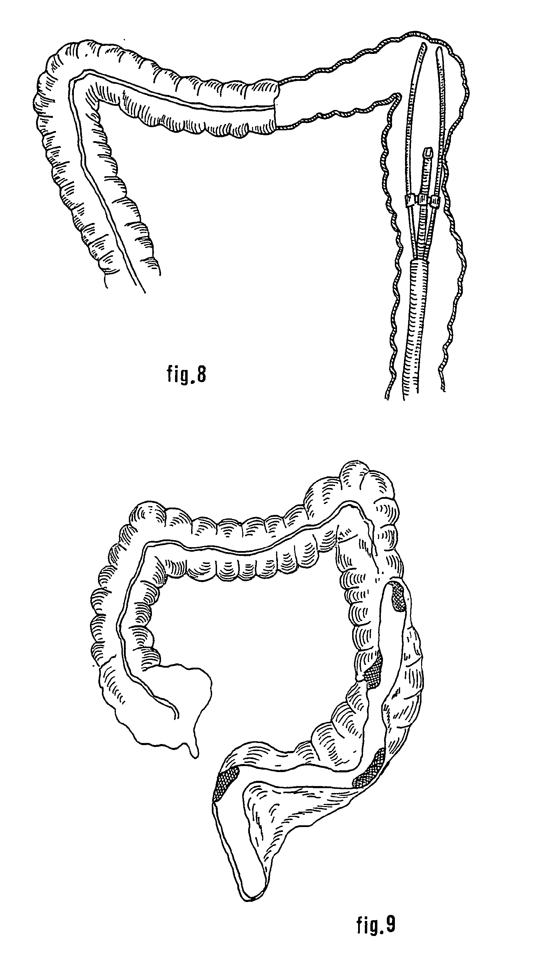

Hence, such mechanisms allows the two working arms to move independently, so as to make them assume complex working configurations, as it is shown e.g. in figures 7 and 8.

The vision device 22 housed in the central body 25 of the endoscope comprises one or more lenses, each thereof being apt to be actuated so as to vary its tilt with respect to the vision device.

According to the preferred embodiment of the present invention, such lenses are two and are tiltable.

Their arrangement onto the central body reunites the features hereto found separately on known endoscopes, i.e. the first lens has a front location whereas the second has a sideways location.

Their tilt, with respect to the vision device, is managed by means for adjusting the position of the lenses, of mechanic type, e.g. comprising a tie rod system operable by the operator, or of electromechanical and/or electronic type, with the aid of magnetostrictive sensors and actuators.

Moreover, the instrument according to the present invention comprises means 40 for processing and visualizing the images taken.

Such means advantageously allows the transfer of the images from the cameras to a computer 40, equipped with specific image reproduction software.

Such processing means are apt to provide stereoscopic images of the area taken and to visualize them, e.g., on a monitor.

This provides the operator with an extremely realistic view of the explored area, as if it were directly observed.

Thus, the operator may use the endoscope in search of the optimum position for operating inside of the organ explored, and not for finding the correct visual angle, as it often happens at present.

Upon finding such optimum position, the combined use of the two independent working arms allows the operator to carry out a large number of surgical interventions.

In fact, the availability of two flexible working arms, together with the central vision device, allows to operate simulating a manual surgical practice, recreating those angles between the two working arms otherwise not viable with common endoscopes equipped with a single working arm.

Moreover, an endoscope according to the present invention may be equipped with means for monitoring its position with respect to the inside of the explored organ.

Such monitoring means comprises one or more signal transmitters positioned on the main body and one or more external receivers of said signals, suitably externally positioned onto the patient's body.

The transmitters could, e.g., be transponders or other devices. The type of receiver will of course depend on the choice of design.

It is understood that such choices do not modify the operation principle of the present invention and should be construed as design choices within the capacity of a person skilled in the art, effected according to the specific embodiment.

The signals received by the receivers provide information on the instrument position inside of the organ.

Such information are advantageously used in order to graphically represent on a monitor the position of the instrument with respect to the explored organ, as it is shown by way of example in figure 9, so as to provide the operator with a real-time checking of the work done.

Moreover, the instrument may also be equipped with means for controlling the frictions between the instrument and the inside walls of the organ.

In particular, such controlling means comprises one or more pressure and/or force sensors, e.g. of membrane and/or of piezoelectric type.

The signals and the information obtained from said sensors are advantageously used to graphically represent the measured parameters on a monitor, providing indications facilitating and improving the operator's work.

The present invention was hereto described according to a preferred embodiment thereof, given by way of example and not for limitative purposes.

It is understood that other embodiments may be provided, all to be construed as falling within the protective scope thereof, as set forth by the annexed claims.

## Claims

1. An endoscopic instrument (10) having a flexible and elongate main body (11) that houses a vision device (22) for taking images of an organ internal area, comprising a first working arm (21) for the use of tools and a second working arm (23) for the use of tools apt to be operated independently with respect to said first working arm (21), **characterized in that** said first and second working arm (21, 23) are transversally movable with respect to said main body (11), moving away and/or nearing the one with respect to the other.

2. The instrument according to claim 1, wherein said main body (11) comprises an annular metal skeleton (12) coated with a rubber material sheath (13).

3. The instrument according to claim 1 or 2, further comprising first handling means of said main body.

4. The instrument according to claim 3, wherein said first handling means comprises one or more tie rods mechanically operated by a user.

5. The instrument according to claim 3 or 4, wherein said first handling means comprises first motion actuating and controlling devices of electronic and/or electromechanical type.

6. The instrument according to any one of the claims 1 to 5, wherein each of said first and second working arm (21, 23) are flexible.

7. The instrument according to any one of the claims 1 to 6, wherein each of said first and second working arm (21, 23) comprises a respective annular metal skeleton coated with a rubber material sheath.

8. The instrument according to any one of the claims 1 a 7, wherein each of said first and second working arm (21, 23) is apt to slide longitudinally with respect to said main body (11), independently the one from the other.

9. The instrument according to any one of the claims 1 to 8, further comprising second handling means of said first and second working arm (21, 23).

10. The instrument according to claim 9, wherein said second handling means comprises one or more tie rods mechanically operated by a user.

11. The instrument according to claim 9 or 10, wherein said second handling means comprises second motion actuating and controlling devices of electronic and/or electromechanical type.

12. The instrument according to any one of the claims 1 to 11, wherein said main body comprises a central body (25), said vision device (22) being connected at the end thereof.

13. The instrument according to claim 12, wherein said first and second working arm (21, 23) are located on two opposite sides of said central body (25).

14. The instrument according to claim 12 or 13, wherein said first and second working arm (21, 23) are connected to said central body by one or more annular mechanisms (30), each of said mechanisms being apt to rotate about said central body (25).

15. The instrument according to claim 14, wherein each of said annular mechanisms (30) is made with an elastically connected mesh structure.

16. The instrument according to any one of the claims 1 to 15, wherein said vision device (22) comprises a camera.

17. The instrument according to claim 16, wherein said camera is of digital type.

18. The instrument according to any one of the claims 1 to 17, wherein said vision device comprises one or more lenses.

19. The instrument according to claim 18, wherein each of said lenses is apt to be handled so as to vary its tilt with respect to the vision device.

20. The instrument according to claim 19, comprising two tiltable lenses.

21. The instrument according to any one of the claims 18 to 20, comprising means for adjusting the position of said lenses.

22. The instrument according to claim 21, wherein said adjusting means are of mechanic type, comprising a tie rod system operable by a user.

23. The instrument according to claim 21, wherein said adjusting means are of electromechanical and/or electronic type.

24. The instrument according to any one of the claims 1 to 23, further comprising means (40) for processing and visualizing the images taken.

25. The instrument according to claim 24, wherein said processing and visualizing means are apt to provide stereoscopic images of the area taken.

26. The instrument according to any one of the claims 1 a 25, further comprising means for monitoring its position with respect to said organ.

27. The instrument according to claim 26, wherein said monitoring means comprises one or more signal transmitters positioned on said main body and one or more external receivers of said signals, said received signals being representative of the position of the instrument.

28. The instrument according to claim 27, wherein said transmitters comprise one or more magnetic field coils.

29. The instrument according to claim 27 or 28, wherein said transmitters comprise one or more transponders.

30. The instrument according to any one of the claims 1 to 29, further comprising means for controlling frictions between the instrument and said organ.

31. The instrument according to claim 30, wherein said means for controlling frictions comprises one or more pressure and/or force sensors.

32. The instrument according to claim 31, wherein one or more of said sensors is of piezoelectric type.

33. The instrument according to claim 31 or 32, wherein one or more of said sensors is of membrane type.

34. The instrument according to any one of the claims 26 to 32, further comprising means for graphically representing said position of the instrument with respect to the organ and said frictions.

## Patentansprüche

1. Endoskopisches Gerät (10) weist einen biegbaren und verlängerten Grundkörper (11) auf, der eine Sichtvorrichtung (22) anordnet, um Bilder eines Bereiches des Organinneren aufzunehmen, welches einen ersten Arbeitsarm (21) für den Einsatz von Werkzeugen, und einen zweiten Arbeitsarm (23) für den Einsatz von Werkzeugen umfasst, der bezüglich des ersten Arbeitsarms (21) geeignet ist, um unabhängig voneinander bedient zu werden,
**dadurch gekennzeichnet, dass**
der erste und der zweite Arbeitsarm (21, 23) quer bezüglich des Grundkörpers (11) beweglich sind, indem sich der eine bezüglich des anderen entfernt und/oder annähert.

2. Gerät gemäß Anspruch 1, worin der Grundkörper (11) ein ringförmiges Metallgerippe (12) umfasst, das mit einer Gummihülle (13) ummantelt ist.

3. Gerät gemäß Anspruch 1 oder 2, darüber hinaus eine erste Bedienungseinrichtung des Grundkörpers umfasst.

4. Gerät gemäß Anspruch 3, worin die erste Bedienungseinrichtung eine oder mehrere Zugstangen umfasst, die mechanisch durch den Anwender bedient werden.

5. Gerät gemäß Anspruch 3 oder 4, worin die erste Bedienungseinrichtung erste elektronische und/oder elektromechanische Bewegungsbedien- und Steuervorrichtungen umfasst.

6. Gerät gemäß jedem der Ansprüche 1 bis 5, worin der erste und der zweite Arbeitsarm (21, 23) biegsam sind.

7. Gerät gemäß jedem der Ansprüche 1 bis 6, worin der erste und der zweite Arbeitsarm (21, 23) ein entsprechendes Metallgerippe umfassen, das mit einer Gummihülle ummantelt ist.

8. Gerät gemäß jedem der Ansprüche 1 bis 7, worin der erste und der zweite Arbeitsarm (21, 23) dazu geeignet sind, unabhängig voneinander in Längsrichtung bezüglich des Grundkörpers (11) zu gleiten.

9. Gerät gemäß jedem der Ansprüche 1 bis 8, darüber hinaus eine zweite Bedieneinrichtung des ersten und des zweiten Arbeitsarms (21, 23) umfasst.

10. Gerät gemäß Anspruch 9, worin die zweite Bedieneinrichtung eine oder mehrere Zugstangen umfasst, die mechanisch durch den Anwender bedient werden.

11. Gerät gemäß Anspruch 9 oder 10, worin die zweite Bedieneinrichtung zweite elektronische und/oder elektromechanische Bewegungsbedien- und Steuervorrichtungen umfasst.

12. Gerät gemäß jedem der Ansprüche 1 bis 11, worin der Grundkörper einen Zentralkörper (25) umfasst, und das Sichtgerät (22) mit dessen Ende verbunden ist.

13. Gerät gemäß Anspruch 12, worin der erste und der zweite Arbeitsarm (21, 23) an zwei gegenüberliegenden Seiten des Zentralkörpers (25) angeordnet sind.

14. Gerät gemäß dem Anspruch 12 oder 13, worin der erste und der zweite Arbeitsarm (21, 23) mit dem Zentralkörper durch eine oder mehrere ringförmige Einrichtungen (30) verbunden sind, und jede dieser Einrichtungen geeignet ist, sich um den Zentralkörper (25) zu drehen.

15. Gerät gemäß dem Anspruch 14, worin jede der ringförmigen Einrichtungen (30) aus einer elastisch verbundenen Netzstruktur hergestellt ist.

16. Gerät gemäß jedem der Ansprüche 1 bis 15, worin das Sichtgerät (22) eine Kamera umfasst.

17. Gerät gemäß Anspruch 16, worin die Kamera digitaler Bauart ist.

18. Gerät gemäß jedem der Ansprüche 1 bis 17, worin das Sichtgerät eine oder mehrere Linsen umfasst.

19. Gerät gemäß Anspruch 18, worin jede der Linsen geeignet ist so bedient zu werden, dass deren Neigung bezüglich des Sichtgerätes verändert werden kann.

20. Gerät gemäß Anspruch 19, welches zwei neigbare Linsen umfasst.

21. Gerät gemäß jedem der Ansprüche 18 bis 20, welches eine Einrichtung zum Einstellen der Position der Linsen umfasst.

22. Gerät gemäß Anspruch 21, worin die Einstelleinrichtung von mechanischer Bauart ist, und ein Zugstangensystem umfasst, das durch den Anwender bedienbar ist.

23. Gerät gemäß Anspruch 21, worin die Einstelleinrichtung von elektromechanischer und/oder von elektronischer Bauart ist.

24. Gerät gemäß jedem der Ansprüche 1 bis 23, umfasst darüber hinaus eine Einrichtung (40) zum Bearbeiten und Darstellen aufgenommener Bilder.

25. Gerät gemäß Anspruch 24, worin die Bearbeitungs- und Darstellungseinrichtung geeignet sind, um Raumbilder vom aufgenommenen Bereich vorzusehen.

26. Gerät gemäß jedem der Ansprüche 1 bis 25, umfasst darüber hinaus eine Einrichtung zum Überwachen seiner Position bezüglich des Organs.

27. Gerät gemäß Anspruch 26, worin die Überwachungseinrichtung ein oder mehrere Signalübertragungssysteme, sowie einen oder mehrere äußere Aufnehmer dieser Signale umfasst, welche auf dem Grundkörper angeordnet sind, und die aufgenommenen Signale darstellend für die Positionen des Gerätes sind.

28. Gerät gemäß Anspruch 27, wobei die Übertragungssysteme eine oder mehrere magnetische Feldspulen umfassen.

29. Gerät gemäß Anspruch 27 oder 28, worin die Übertragungssysteme eine oder mehrere Umsetzer umfassen.

30. Gerät gemäß jedem der Ansprüche 1 bis 29, umfasst darüber hinaus eine Einrichtung zum Steuern von Spannungen zwischen dem Gerät und dem Organ.

31. Gerät gemäß Anspruch 30, worin die Einrichtung zum Steuern von Spannungen einen oder mehrere Druck- und/oder Kraftmessfühler umfasst.

32. Gerät gemäß Anspruch 31, worin einer oder mehrere Messfühler von piezoelektrischer Bauart sind.

33. Gerät gemäß Anspruch 31 oder 32, worin einer oder mehrere Messfühler von membranartiger Bauart sind.

34. Gerät gemäß jedem der Ansprüche 26 bis 32, umfasst darüber hinaus eine Einrichtung zum graphischen Darstellen der Position des Gerätes bezüglich des Organs und der Spannungen.

## Revendications

1. Instrument endoscopique (10) doté d'un corps principal flexible et allongé (11) qui abrite un dispositif de vision (22) pour prendre des images d'une région interne d'organe, comportant un premier bras de travail (21) pour l'utilisation d'outils et un second bras de travail (23) pour l'utilisation d'outils, susceptible d'être commandé indépendamment dudit premier bras de travail (21), **caractérisé en ce que** lesdits premier et second bras de travail (21, 23) sont mobiles transversalement par rapport audit corps principal (11), en s'écartant et/ou en se rapprochant l'un de l'autre.

2. Instrument selon la revendication 1, dans lequel ledit corps principal (11) comporte un squelette métallique annulaire (12) recouvert d'une gaine constituée de caoutchouc (13).

3. Instrument selon la revendication 1 ou 2, comportant en outre un premier moyen de manipulation dudit corps principal.

4. Instrument selon la revendication 3, dans lequel ledit premier moyen de manipulation comporte un ou plusieurs tirants commandés mécaniquement par un utilisateur.

5. Instrument selon la revendication 3 ou 4, dans lequel ledit premier moyen de manipulation comporte des premiers dispositifs de déclenchement et de commande de mouvements de type électronique et/ou électromécanique.

6. Instrument selon l'une quelconque des revendications 1 à 5, dans lequel chacun desdits premier et second bras de travail (21, 23) est flexible.

7. Instrument selon l'une quelconque des revendications 1 à 6, dans lequel chacun desdits premier et second bras de travail (21, 23) comporte un squelette métallique annulaire respectif recouvert d'une gaine constituée de caoutchouc.

8. Instrument selon l'une quelconque des revendications 1 à 7, dans lequel chacun desdits premier et second bras de travail (21, 23) est apte à glisser longitudinalement par rapport audit corps principal (11), indépendamment de l'autre.

9. Instrument selon l'une quelconque des revendications 1 à 8, comportant en outre un second moyen de manipulation desdits premier et second bras de travail (21,23).

10. Instrument selon la revendication 9, dans lequel ledit second moyen de manipulation comporte un ou plusieurs tirants commandés mécaniquement par un utilisateur.

11. Instrument selon la revendication 9 ou 10, dans lequel ledit second moyen de manipulation comporte des seconds dispositifs de déclenchement et de commande de mouvements de type électronique et/ou électromécanique.

12. Instrument selon l'une quelconque des revendications 1 à 11, dans lequel ledit corps principal comporte un corps central (25), ledit dispositif de vision (22) étant relié à l'extrémité de celui-ci.

13. Instrument selon la revendication 12, dans lequel lesdits premier et second bras de travail (21, 23) sont situés sur deux côtés opposés dudit corps central (25).

14. Instrument selon la revendication 12 ou 13, dans lequel lesdits premier et second bras de travail (21,23) sont reliés audit corps central par un ou plusieurs mécanismes annulaires (30), chacun desdits mécanismes étant aptes à tourner autour dudit corps central (25).

15. Instrument selon la revendication 14, dans lequel chacun desdits mécanismes annulaires (30) est constitué d'une structure maillée à liaison élastique.

16. Instrument selon l'une quelconque des revendications 1 à 15, dans lequel ledit dispositif de vision (22) comporte une caméra.

17. Instrument selon la revendication 16, dans lequel ladite caméra est de type numérique.

18. Instrument selon l'une quelconque des revendications 1 à 17, dans lequel ledit dispositif de vision comporte une ou plusieurs lentilles.

19. Instrument selon la revendication 18, dans lequel chacune desdites lentilles est apte à être manipulée afin de faire varier son inclinaison par rapport au dispositif de vision.

20. Instrument selon la revendication 19, comportant deux lentilles inclinables.

21. Instrument selon l'une quelconque des revendications 18 à 20, comportant des moyens permettant de régler la position desdites lentilles.

22. Instrument selon la revendication 21, dans lequel lesdits moyens de réglage sont de type mécanique, et comportent un système de tirants susceptible d'être commandé par un utilisateur.

23. Instrument selon la revendication 21, dans lequel lesdits moyens de réglage sont de type électromécanique et/ou électronique.

24. Instrument selon l'une quelconque des revendications 1 à 23, comportant en outre des moyens (40) pour traiter et visualiser les images prises.

25. Instrument selon la revendication 24, dans lequel lesdits moyens de traitement et de visualisation sont aptes à fournir des images stéréoscopiques de la zone prise.

26. Instrument selon l'une quelconque des revendications 1 à 25, comportant en outre un moyen pour surveiller sa position par rapport audit organe.

27. Instrument selon la revendication 26, dans lequel ledit moyen de surveillance comporte un ou plusieurs émetteurs de signal positionnés sur ledit corps principal et un ou plusieurs récepteurs externes desdits signaux, lesdits signaux reçus étant représentatifs de la position de l'instrument.

28. Instrument selon la revendication 27, dans lequel lesdits émetteurs comportent une ou plusieurs bobines à champ magnétique.

29. Instrument selon la revendication 27 ou 28, dans lequel lesdits émetteurs comportent un ou plusieurs transpondeurs.

30. Instrument selon l'une quelconque des revendications 1 à 29, comportant en outre un moyen pour contrôler les frictions entre l'instrument et ledit organe.

31. Instrument selon la revendication 30, dans lequel ledit moyen de contrôle des frictions comporte un ou plusieurs capteurs de pression et/ou de force.

32. Instrument selon la revendication 31, dans lequel les un ou plusieurs dits capteurs sont de type piézoélectrique.

33. Instrument selon la revendication 31 ou 32, dans lequel les un ou plusieurs dits capteurs sont de type à membrane.

34. Instrument selon l'une quelconque des revendications 26 à 32, comportant en outre un moyen pour représenter de manière graphique ladite position de l'instrument par rapport à l'organe et lesdites frictions.
